# EUROPEAN PATENT APPLICATION

(11) **EP 4 201 426 A1**
(43) Date of publication of application: **28.06.2023**
(21) Application number: 21383214.0
(22) Date of filing: 23.12.2021
(51) Int. Cl.: A61K 45/06, A61K 31/4406, A61K 31/519, A61P 35/00, A61P 35/02

(54) **PHARMACEUTICAL COMPOSITIONS FOR INDUCING HDAC7 EXPRESSION**

(71) Applicant: Fundació Institut de Recerca Contra la Leucèmia Josep Carreras, 08916 Barcelona (ES)
(72) Inventor: PARRA BOLA, María Isabel, 08916 Badalona (ES); DE BARRIOS BARRI, Oriol, 08916 Badalona (ES)
(74) Representative: Hoffmann Eitle

(57) **Abstract**

The invention relates to compositions for use in a method of inducing expression of HDAC7 in malignant hematologic cells, the compositions comprising at least one Menin inhibitor and optionally a Class I HDAC inhibitor. The invention further relates to a kit of parts comprising at least two recipients, wherein one recipient comprises at least one Menin inhibitor and the other recipient comprises at least one Class I HDAC inhibitor. The invention further relates to the compositions as described herein for use in a method of treatment of B cell acute lymphoblastic leukemia (B-ALL), diffuse large B cell lymphoma (DLBCL), follicular lymphoma (FL) or T-cell acute lymphoblastic leukemia (T-ALL).

## Description

### Technical field

The invention relates to compositions for use in a method of inducing expression of HDAC7 in malignant hematologic cells, the compositions comprising at least one Menin inhibitor and optionally a Class I HDAC inhibitor. The invention further relates to a kit of parts comprising at least two recipients, wherein one recipient comprises at least one Menin inhibitor and the other recipient comprises at least one Class I HDAC inhibitor. The invention further relates to the compositions as described herein for use in a method of treatment of B cell acute lymphoblastic leukemia (B-ALL), diffuse large B cell lymphoma (DLBCL), follicular lymphoma (FL), or T-cell acute lymphoblastic leukemia (T-ALL).

### Background of the invention

Acute lymphoblastic leukemia derived from B cell progenitors (pro-B cells) (pro-B-ALL) is the most prevalent type of malignancy among the 350 new infants that are annually diagnosed of leukemia in Spain. Despite the fact that 80% of B-ALL remission has been achieved due to recent therapeutic advances, a specific subgroup of patients that present the t(4;11) rearrangement commonly relapse, converting acute leukemia into the main cause of pediatric cancer-associated death. Current therapy in this group of patients consists of an initial phase that includes prednisone and L-asparaginase, followed by an induction stage with cytarabine, vincristine and daunorubicin, since it is believed that leukemic cells may derive from a myeloid progenitor. However, survival rate of infants (< 1 year old) diagnosed with t(4;11)-rearranged pro-B-ALL does not even reach 35%. In fact, due to their young age and vulnerability, these infants are normally excluded from clinical trials. There is, thus, an urgent need of improving current therapy and implementing novel combinatorial treatments and precision medicine.

The generation of properly differentiated hematopoietic cell populations is one of the major focuses of research on leukemia. In this area, the function of key lineage-specific transcription factors that promote cell differentiation processes is well established. However, increasing evidence demonstrates that lymphocyte-specific factors not only induce the expression of B-cell specific genes, but they also lead to the repression of lineage-inappropriate genes ensuring the correct identity of B lymphocytes. Therefore, the aberrant expression and/or mutation of many of these factors involved in B lymphocyte development have been linked to the outcome of hematopoietic malignancies, such as pro-B-ALL. For instance, overexpression of the transcription factor c-MYC is known to be involved in B-ALL and other type of hematological malignancies derived from B lymphocytes.

During terminal differentiation in peripheral lymphoid organs, B cells generate a hugely diverse repertoire of antibodies, which enable specific immune responses against pathogens that the organism may encounter. The generation of the antibodies by mature B cells after facing the antigen takes place in the germinal centers (GCs). As it occurs during earlier stages of B cell development, an aberrant regulation of key transcription factors at this point also triggers the onset of hematological malignancies, such as diffuse large B cell lymphoma (DLBCL) or follicular lymphoma (FL). Both DLBCL and FL are frequent types of non-Hodgkin lymphoma (NHL). On the one hand, FL is an indolent and slow-growing form of lymphoma, while DLBCL is the most aggressive type of NHL worldwide, accounting for a total of 40% of all NHL. DLBCL presents large genetic and clinical heterogeneity and a significant subset relapse after regular chemotherapeutic treatment, which associates with decreased survival.

Histone or protein deacetylases (HDACs) are epigenetic regulators that have emerged as crucial transcriptional repressors in highly diverse physiological and pathological systems. To date, 18 human HDACs have been identified and grouped into four classes: Class I HDACs (HDAC1, 2, 3, and 8), class II HDACs (HDAC4, 5, 6, 7, 9, and 10), class III HDACs, also called sirtuins (SIRT1, 2, 3, 4, 5, 6, and 7), and class IV HDAC (HDAC11). Class II HDACs are further subdivided into class IIa (HDAC4, 5, 7, 9) and class IIb (HDAC6 and 10) groups. The emergence of HDACs as regulators at diverse steps of immune system differentiation has set them as potential candidates of therapeutic strategies aiming to treat childhood leukemias. Mutation and/or abnormal expression of class IIa HDACs have been frequently observed in disease, particularly in cancer, making them important therapeutic targets. Consequently, histone deacetylase inhibitors (HDIs) have become promising therapeutic agents.

However, we are far from fully understanding the contribution of individual HDACs to cancer. In fact, some HDACs may be under expressed or present inactivating mutations. Up to date, the approval of HDAC inhibitors has been restricted to pan-HDAC inhibitors for the treatment of T-cell malignancies and multiple myeloma. However, given the potential function of HDAC members like HDAC7 in pro-B-ALL, a global repression of all HDAC subclasses is potentially detrimental for patients. In this sense, and in an attempt to avoid the harmful effects of inhibition of all HDACs, new drugs with selective HDAC subclass inhibition are being developed.

In this sense, subclass I inhibitor Chidamide, that has been already tested in MLL-rearranged hematological disorders, appears to be a promising candidate for pro-B-ALL treatment, since it inhibits HDAC1-3 (Histone protein deacetylase 1-3 (HDAC1-3) without affecting HDAC7 activity.

The histone deacetylase HDAC7 has been identified as a novel biomarker that determines prognosis in t(4;11) pro-B-ALL patients (de Barrios O et al. Leukemia, 2021). De Barrios et al. provide data that strongly supports the use of HDAC7 expression levels as a prognostic marker in infant pro-B-ALL with t(4;11) rearrangement. Data has been obtained in primary samples from pro-B-ALL diagnosed infants and cell lines with t(4;11) translocation, such as SEM-K2. This cell line is derived from peripheral blood of a 5-year-old female at relapse. HDAC7 expression was significantly decreased in this cell line, as well as in infants with t(4;11) pro-B-ALL, when compared to samples from pediatric patients with an alternative rearrangement, such as t(9;11), or an unaltered *KMT2A* gene, which encodes the MLL protein (Pieters R et al. Lancet, 2007; 370:240-50).

When combining data from all subtypes of leukemia, patients displaying low or intermediate levels of HDAC7 present the poorest survival. In consequence, the absence of HDAC7 seems to be a key element in determining the poor outcome associated to t(4;11) infant pro-B-ALL.

It has also been shown that HDAC7 is not only under expressed in pro-B-ALL cells, but that these cells need to maintain HDAC7 at very low levels of expression, in order to maintain an aberrantly active proliferation rate (de Barrios O et al. Leukemia, 2021). In this publication the authors also found that key cancer hallmarks such as cellular differentiation and apoptosis were altered. For instance, the pro-apoptotic marker MMP9 (Yadav et al. Cell Death Dis 2020) and the marker of proper B cell differentiation CD86 (Engel et al. Blood 1994) were triggered by the induction of HDAC7. Oppositely, asparagine synthetase (ASNS) was downregulated in SEM-K2 cells expressing HDAC7. This gene is associated to a worse prognosis in pro-B-ALL, since it blocks the early response to state-of-the-art treatment with L-asparaginase. The authors also found that t(4;11) infants with the lowest expression of HDAC7 displayed significantly increased levels of the chemoresistance marker ASNS, indicating that lack of HDAC7 implies an increased resistance to standard treatment.

Therefore, it can be concluded that a forced induction of HDAC7 in this highly aggressive subtype of pro-B-ALL prevents the uncontrolled proliferation of malignant cells and confers them a higher sensitivity to current chemotherapy.

The current working hypothesis is therefore that the restoration of HDAC7 expression constitutes a promising therapeutic strategy in terms of improving survival among t(4;11) pro-B-ALL patients since it reduces the leukemogenic capacity of the cells.

It was, therefore, a target of present invention to identify a method of increasing the expression of HDAC7 in malignant hematologic cells and to provide pharmaceutical compositions that can be administered to patients suffering from pro-B-ALL that are capable of increasing the expression of HDAC7. It was a further goal of present invention to identify a combinatorial precision therapy that in addition to inducing HDAC7 expression, can enhance its leukemia suppressor function.

### BRIEF DESCRIPTION OF THE FIGURES

Figure 1: Use of Menin inhibitor MI-538 and class I HDAC selective inhibitor Chidamide to trigger HDAC7 expression in t(4;11) pro-B-ALL cell lines. (A) Protein levels of HDAC7 in SEM-K2 and RS4;11 cells. (B) qRT-PCR for HDAC7 in SEM-K2 cells treated with MI-538 (1 µM, 6 days) Chidamide (1 µM, 48 hours), or DMSO as control. (C) Relative protein expression of HDAC7 in SEM-K2 and RS4;11 cells.
Figure 2: Use of MI-538 and Chidamide in combination exerts a surprising effect in reducing t(4;11) pro-B-ALL cells viability. (A) Protein levels of HDAC7 in SEM-K2 and RS4;11 cells (t(4;11) translocation); and REH cell line (no chromosomal alteration at KMT2A gene). β-actin was used as loading control. (B) MTT assay to assess cell viability in SEM-K2, RS4;11 and REH cell lines treated with MI-538 (1 µM, 6 days) and/or Chidamide (1 µM, 48 hours), or DMSO as control.
Figure 3: Synergistic induction of HDAC7 after combinatorial therapy with MI-538 and Chidamide alters the expression of key HDAC7 targets. qRT-PCR was performed for MMP9 (involved in apoptosis), CD86 (marker of proper B lymphocytes differentiation) and ASNS (chemoresistance marker in infant leukemia) in SEM-K2 cells treated with MI-538 (1 µM, 6 days) and/or Chidamide (1 µM, 48 hours), or DMSO as control.
Figure 4: Effect of HDAC7 expression on the regular and standard therapy that is commonly used in clinical settings in infant t(4;11) pro-B-ALL patients. SEM-TetOn-Tight-HDAC7 cell line was induced with doxycycline. As shown in Figure 4, HDAC7 induction endows SEM-K2 cells with a higher sensitivity to prednisone treatment, since the reduction of viability is significant at a 10-time lower dose than in normal conditions (i.e. with low HDAC7 levels).
Figure 5: MTT assay to assess cell viability in SEM-K2 and REH cell lines treated with MI-503 (1 µM, 6 days) and/or Chidamide (1 µM, 48 hours), or DMSO as control.
Figure 6: MTT assay to assess cell viability in SEM-K2 and REH cell lines treated with MI-463 (1 µM, 6 days) and/or Chidamide (1 µM, 48 hours), or DMSO as control.
Figure 7: MTT assay to assess cell viability in SEM-K2 and REH cell lines treated with MI-3454 (1 µM, 6 days) and/or Chidamide (1 µM, 48 hours), or DMSO as control.
Figure 8: MTT assay to assess cell viability in RS4;11 cell lines treated with MI-503, MI-463 or MI-3454 (1 µM, 6 days) and/or Chidamide (1 µM, 48 hours), or DMSO as control.
Figure 9: qRT-PCR for HDAC7 in RS4;11 cells treated with MI-503, MI-463 or MI-3454 (1µM, 6 days) and/or Chidamide (1 µM, 48 hours).
Figure 10: Protein levels of HDAC7 in SEM-K2 cells treated with MI-503 or MI-463 (1µM, 6 days) and/or Chidamide (1 µM, 48 hours), or DMSO as control.

### SUMMARY OF THE INVENTION

The present invention relates to a composition, preferably a pharmaceutical composition, for use in a method of inducing expression of HDAC7 in malignant hematologic cells, wherein the composition comprises at least one Menin inhibitor.

In one embodiment of present invention the at least one Menin inhibitor is a compound according to formula (A) 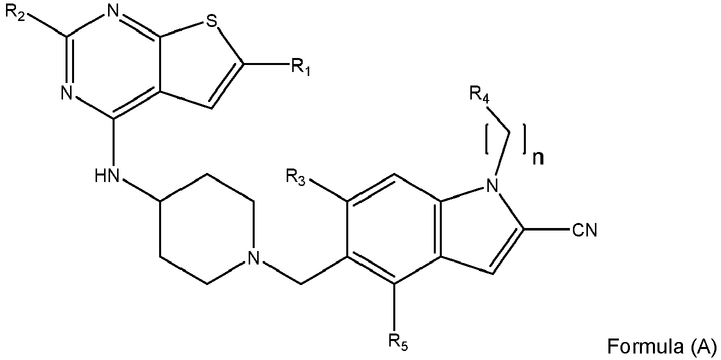 wherein
R₁ is a C₁₋₄ alkyl group including branched alkyl optionally substituted with up to three halogen atoms;
R₂ is H or NRaRb, Ra and Rb are independently H or C₁₋₄ alkyl;
R₃ is H or OH;
R₄ independently represents H, C₁₋₄ alkyl group including cycloalkyl, or aryl group including heteroaryl.
R₄ is optionally substituted with R₆, where R₆ represents H, NRcRd and Rc and Rd are independently H, C₁₋₄ alkyl group including branched alkyl optionally substituted with up to three halogen atoms, CHO or CONRcRd
n is 0 - 4
R₅ is H or a C₁₋₄ alkyl group including branched alkyl optionally substituted with up to three halogen atoms.

In a further aspect the at least one Menin inhibitor is a compound according to formula (A), wherein
R₁ is -CH₂CF₃
R₂ is H or MeNH-
R₃ is H or OH
R₄ independently represents H, a 4-pyrazolyl group (I) or a substituted bicyclic alkyl group (II) 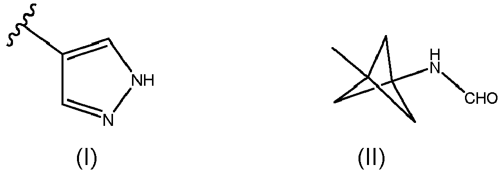
n is 0 - 4
R₅ is H or methyl.

In a preferred aspect the at least one Menin inhibitor is a compound according to formula (A), wherein
R₁ is -CH₂CF₃
R₂ is H
R₃ is H or OH
R₄ is H or a 4-pyrazolyl group - 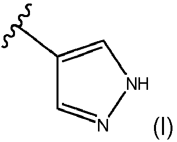
n is 0 or 1
R₅ is H or methyl.

In an even more preferred aspect, the at least one Menin inhibitor is a compound according to formula (A), wherein
R₁ is -CH₂CF₃
R₂ and R₅ are H
R₃ is OH
R₄ is a 4-pyrazolyl group - 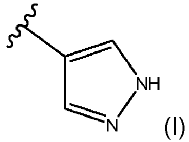
n is 1.

In one embodiment of the present invention the Menin inhibitor is selected from the group consisting of MI-538, MI-503, MI-463, MI-3454, MI-1481 or MI-2, or a combination thereof. Preferably the Menin inhibitor of present invention is selected from MI-538, MI-503, MI-463 or MI-3454, most preferred is MI-538.

In a further embodiment of present invention, the expression of HDAC7 is mRNA expression and said mRNA expression of HDAC7 is increased by at least about 1.2-fold, 1.3-fold, 1.4-fold, 1.5-fold in the malignant hematologic cells compared to the mRNA expression of HDAC7 in reference cells.

In yet a further embodiment of present invention the expression of HDAC7 is protein expression and said protein expression of HDAC7 is increased by at least about 1.5-fold, 2.0-fold, 2.5-fold, 3.0-fold, 3.5-fold, 4.0-fold, 4.5-fold, 5.0-fold, 5.5-fold, 6.0-fold, 6.5-fold, 7.0-fold, 7.5-fold, 8.0-fold, 8.5-fold, 9.0-fold, 9.5-fold, 10.0-fold, 10.5-fold, 11.0-fold, 11.5-fold, 12.0-fold in the malignant hematologic cells compared to the protein expression of HDAC7 in reference cells.

In one embodiment of present invention the malignant hematologic cells are selected from B cell acute lymphoblastic leukemia (B-ALL) cells, diffuse large B cell lymphoma (DLBCL) cells, follicular lymphoma (FL) and T-cell acute lymphoblastic leukemia (T-ALL) cells. In a preferred embodiment the B-ALL cells are B cell acute lymphoblastic leukemia (B-ALL) cells, in the most preferred embodiment the B-ALL cells pro-B-ALL cells characterized by a t(4;11) rearrangement.

In a further embodiment of present invention, the composition further comprises at least one Class I HDAC inhibitor. The at least one Class I HDAC inhibitor can be selected from the group consisting of Chidamide, Entinostat, Mocetinostat, Tacedinaline or Romidepsin, or a combination thereof. A preferred Class I HDAC inhibitor of present invention is Chidamide.

In one embodiment of present invention the at least one Menin inhibitor is MI-538 and the at least one Class I HDAC inhibitor is Chidamide.

The present invention further relates to a kit of parts comprising at least two recipients, wherein one recipient comprises at least one Menin inhibitor and the other recipient comprises at least one Class I HDAC inhibitor.

In one embodiment the kit of parts comprises at least two recipients, wherein one recipient comprises at least one Menin inhibitor as described herein, preferably said Menin inhibitor is selected from the group consisting of MI-538, MI-503, MI-463, MI-3454, MI-1481 or MI-2, or a combination thereof, more preferred the group consisting of MI-538, MI-503, MI-463 and MI-3454, most preferred the Menin inhibitor being MI-538, and the other recipient comprises at least one Class I HDAC inhibitor selected from the group consisting of Chidamide, Entinostat, Mocetinostat, Tacedinaline or Romidepsin, preferably wherein the at least one Class I HDAC inhibitor is Chidamide.

In one preferred embodiment of present invention the kit of parts comprises at least two recipients, wherein one recipient comprises MI-538 and the other recipient comprises Chidamide.

The present invention furthermore relates to the composition as disclosed herein above for use in a method of treatment of B cell acute lymphoblastic leukemia (B-ALL), diffuse large B cell lymphoma (DLBCL), follicular lymphoma (FL) or T-cell acute lymphoblastic leukemia (T-ALL), preferably of B cell acute lymphoblastic leukemia (B-ALL).

The present invention furthermore relates to a method for treating B cell acute lymphoblastic leukemia (B-ALL), diffuse large B cell lymphoma (DLBCL), follicular lymphoma (FL) or T-cell acute lymphoblastic leukemia (T-ALL), preferably B cell acute lymphoblastic leukemia (B-ALL) in a subject in need thereof, comprising administering to said subject the composition as defined herein above.

### DETAILED DESCRIPTION OF THE INVENTION

The present invention may be understood more readily by reference to the following detailed description of the preferred embodiments of the invention, and to the examples included therein.

The restoration of HDAC7 expression constitutes a promising therapeutic strategy in terms of improving survival among t(4;11) pro-B-ALL patients since it reduces the leukemogenic capacity of the cells. It was therefore a target of present invention to identify a method of increasing the expression of HDAC7 in malignant hematologic cells and to provide pharmaceutical compositions that can be administered to patients suffering from pro-B-ALL that are capable of increasing the expression of HDAC7. It was a further goal of present invention to identify a combinatorial precision therapy that, in addition to inducing HDAC7 expression, can enhance its leukemia suppressor function.

The present invention therefore relates to a composition, preferably a pharmaceutical composition, for use in a method of inducing expression of HDAC7 in malignant hematologic cells, wherein the composition comprises at least one Menin inhibitor.

Menin inhibitors are novel targeted agents currently in clinical development for the treatment of genetically defined subsets of acute leukemia. Menin has a tumor suppressor function in endocrine glands. Germline mutations in the gene encoding Menin cause the multiple endocrine neoplasia type 1 (MEN1) syndrome, a hereditary condition associated with tumors of the endocrine glands. However, Menin is also critical for leukemogenesis in subsets driven by rearrangement of the Lysine Methyltransferase 2A (*KMT2A*) gene, also known as mixed lineage leukemia (MLL), which encodes an epigenetic modifier.

Whilst Menin inhibitors and their various uses have been known for some time, their effect on the expression of HDAC7 in malignant hematologic cells has not been previously described. The inventors have now surprisingly found that Menin inhibitors can induce the expression of HDAC7 in such cancer cells, thus providing a potential new treatment of diseases caused by reduced cellular HDAC7 expression, such as B cell acute lymphoblastic leukemia (B-ALL), diffuse large B cell lymphoma (DLBCL), follicular lymphoma (FL) or T-cell acute lymphoblastic leukemia (T-ALL).

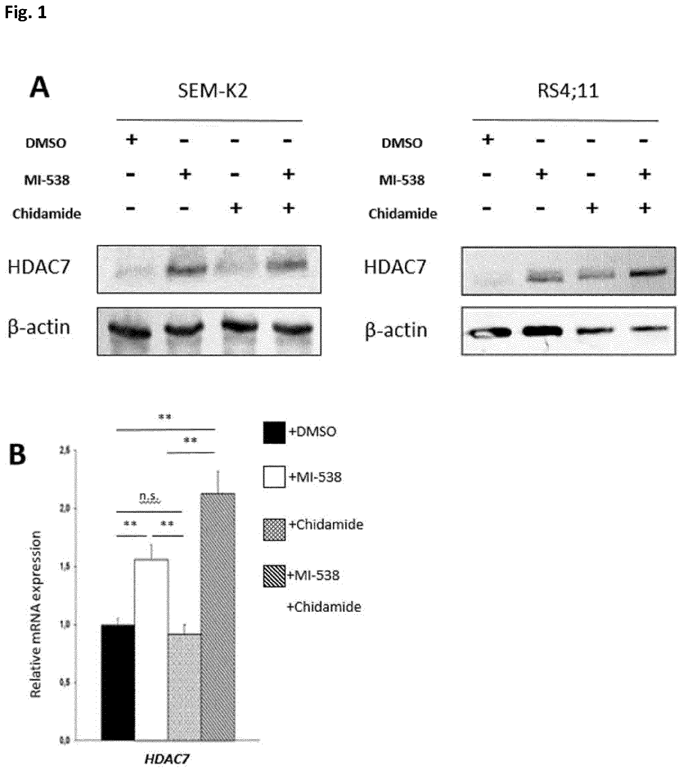

The inventors have used SEM-K2 and RS4;11 cell lines harboring t(4;11) translocation and have shown that when incubated with the Menin inhibitor MI-538, a clear upregulation of HDAC7 at protein levels in both cell lines (Figure 1A) and also at mRNA level in SEM-K2 cells (Figure 1B) could be observed (see Example 1). In order to enhance the effect of the Menin inhibitor the inventors added the class I HDAC selective inhibitor Chidamide. As can be seen from Figure 1B, in SEM-K2 cells, treatment with Chidamide alone does not provide any increase in mRNA expression of HDAC7, whereas treatment with MI-538 alone leads to an about 1.5-fold increase of mRNA expression of HDAC7 in the cells. Surprisingly, the combination of Chidamide and Menin inhibitor MI-538 provides a significant and synergistic increase in comparison to the use of Chidamide or MI-538 alone in SEM-K2 cells.

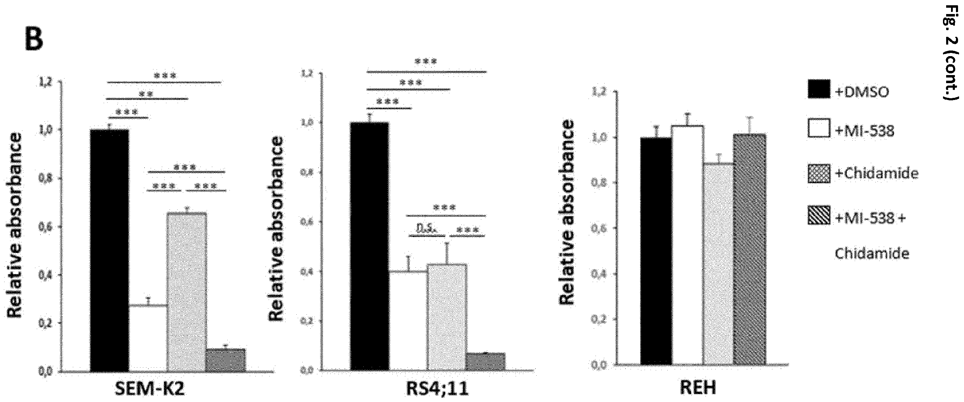

The increase of the mRNA expression of HDAC7 of at least 1.2-fold will already show an effect on the cell viability. The increase of about 1.5-fold as for Menin inhibitor MI-538 is therefore striking and leads to the strong effects on the cell viability when MI-538 is used alone and/or in combination with Chidamide as shown in Fig.2B.

In a further embodiment of present invention, in which the expression of HDAC7 refers to the mRNA expression, said mRNA expression of HDAC7 is increased by at least about 1.2-fold, 1.3-fold, 1.4-fold, 1.5-fold in the malignant hematologic cells compared to the mRNA expression of HDAC7 in reference cells. It is to be understood that the reference cells refer to the same type of malignant hematologic cells as the ones being assessed for their increase in mRNA expression of HDAC7, but which have not been treated with the composition as disclosed herein.

As could be shown in the cell viability assays performed by the inventors and as shown in the Examples, such an increase of HDAC7 mRNA expression leads to decreased cell viability in the malignant hematologic cells.

In yet a further embodiment of present invention the expression of HDAC7 is protein expression and said protein expression of HDAC7 is increased by at least about 1.5-fold, 2.0-fold, 2.5-fold, 3.0-fold, 3.5-fold, 4.0-fold, 4.5-fold, 5.0-fold, 5.5-fold, 6.0-fold, 6.5-fold, 7.0-fold, 7.5-fold, 8.0-fold, 8.5-fold, 9.0-fold, 9.5-fold, 10.0-fold, 10.5-fold, 11.0-fold, 11.5-fold, 12.0-fold in reference cells. It is to be understood that the reference cells refer to the same type of malignant hematologic cells as the ones being assessed for their increase in protein expression of HDAC7, but which have not been treated with the composition as disclosed herein.

In one embodiment of present invention the malignant hematologic cells are selected from B cell acute lymphoblastic leukemia (B-ALL) cells, diffuse large B cell lymphoma (DLBCL) cells, follicular lymphoma (FL) and T-cell acute lymphoblastic leukemia (T-ALL) cells. In a preferred embodiment the B-ALL cells are B cell acute lymphoblastic leukemia (B-ALL) cells, in the most preferred embodiment the B-ALL cells pro-B-ALL cells characterized by a t(4;11) rearrangement.

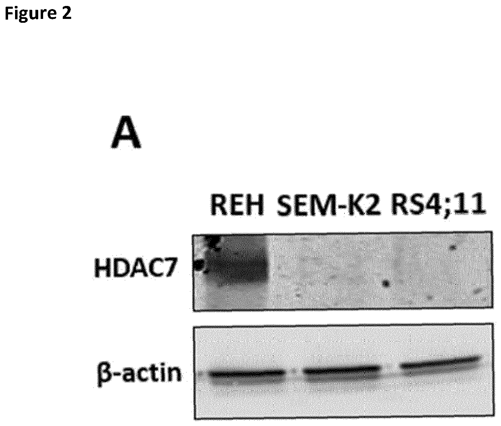

MTT viability assays including previously used SEM-K2 and RS4;11 cells, together with REH cells, that harbor an unaltered form of MLL gene, showed that REH cells displayed abundant levels of HDAC7, compared to t(4;11) translocated cells (Figure 2A) and that MI-538 strongly reduced viability of both SEM-K2 and RS4;11 cells (Example 2). Strikingly, the combination with Chidamide produced an even more dramatic effect, being significantly stronger than both drugs administered alone (Figure 2B, left and center panels). The demonstration that the effect of these drugs is, at least partially, a consequence of HDAC7 induction can be extracted from the MTT assays performed in REH cell line. This cell line, with strong expression of HDAC7, did not respond to MI-538 and/or Chidamide treatments, and no effect was observed on cell viability (Figure 2B, right panel).

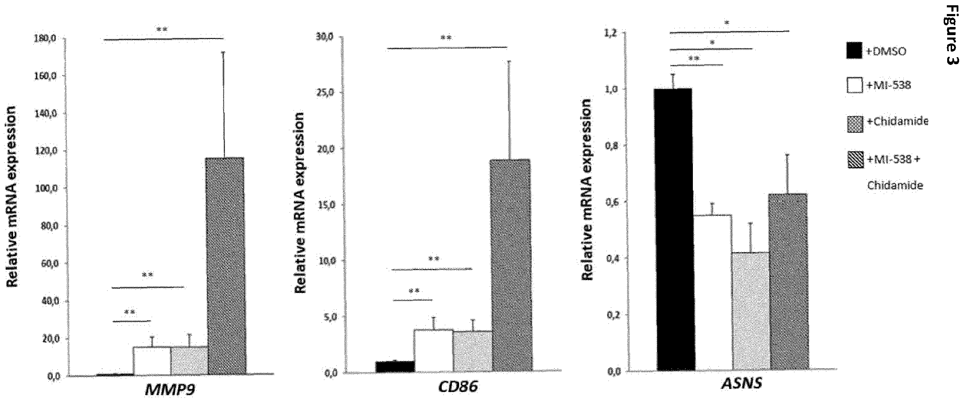

A forced overexpression of HDAC7 in SEM-K2 shifts the genomic profile of t(4;11) pro-B-ALL cells towards a more anti-oncogenic profile through the altered expression of targets involved in key hallmarks of cancer, such as cell differentiation, chemotherapy resistance or apoptosis prevention. In order to corroborate whether the effect of MI-538 and Chidamide on endogenous HDAC7 can be paralleled to that of exogenous HDAC7 induction, some of HDAC7's previously reported targets were analyzed (Example 3). It could be shown that, as expected, both MI-538 and Chidamide triggered the mRNA levels of the pro-apoptotic gene MMP9 and the marker of B lymphocyte differentiation CD86, showing a synergistic effect when both drugs were administered together (Figure 3, left and center panels). Oppositely, the chemoresistance marker ASNS is downregulated upon treatment of SEM-K2 cells with MI-538 and Chidamide (Figure 3, right panel), according to previous data showing an opposite pattern of expression with HDAC7 (de Barrios et al. 2021). Therefore, the effect of MI-538 and Chidamide not only affects HDAC7 itself, but also its previously described targets, reinforcing the potential use of this combinatorial therapy to improve outcome of t(4;11) pro-B-ALL.

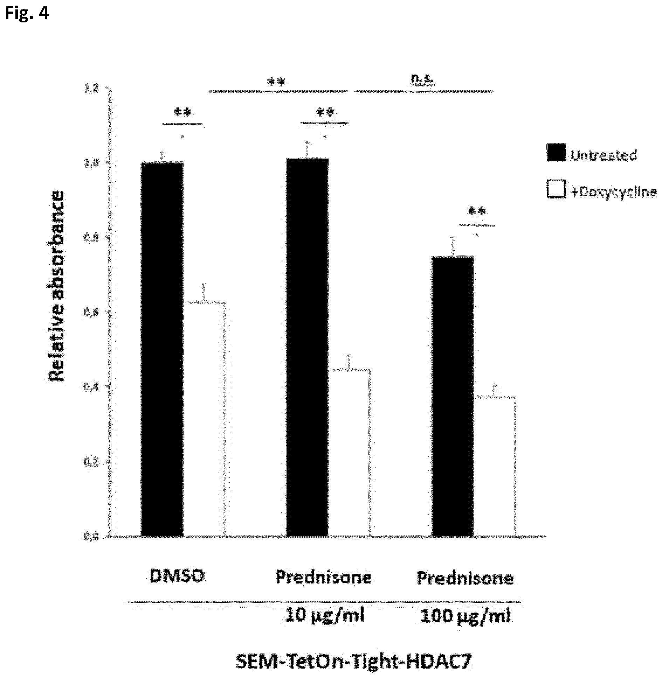

As a proof-of-concept, the effect of HDAC7 expression was evaluated on the regular and standard therapy that is commonly used in clinical settings in infant t(4;11) pro-B-ALL patients. SEM-TetOn-Tight-HDAC7 cell line (also used in de Barrios et al. 2021), that permits HDAC7 induction with doxycycline treatment was used. As shown in Figure 4, HDAC7 induction endows SEM-K2 cells with a higher sensitivity to prednisone treatment, since the reduction of viability is significant at a 10-time lower dose than in normal conditions (i.e. with low HDAC7 levels).

In order to asses if this surprising effect can also be observed with other Menin inhibitors, the inventors have tested further compounds, as shown in Examples 5 to 10. As can be seen from the figures, all tested Menin inhibitors MI-3454, MI-503 and MI-463 reduce viability of SEM-K2 and RS4;11 cells. The combination with Chidamide enhanced the effect. As was expected, an upregulation of HDAC7 mRNA as well as protein expression could be shown in SEM-K2 cells, which was in line with the above findings for Menin inhibitor MI-538.

In one embodiment of present invention the Menin inhibitor is therefore a compound according to formula (A) 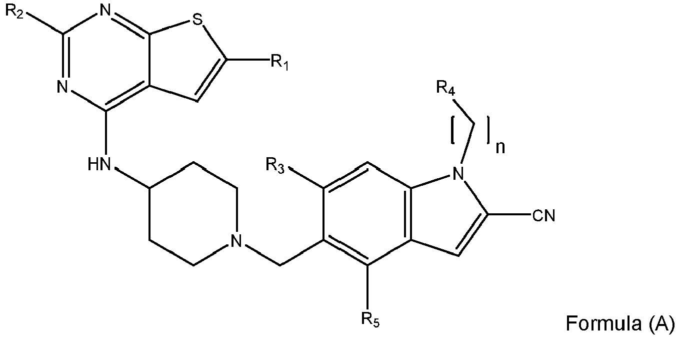 wherein
R₁ is a C₁₋₄ alkyl group including branched alkyl optionally substituted with up to three halogen atoms;
R₂ is H or NRaRb, Ra and Rb are independently H or C₁₋₄ alkyl;
R₃ is H or OH;
R₄ independently represents H, C₁₋₄ alkyl group including cycloalkyl, or aryl group including heteroaryl;
R₄ is optionally substituted with R₆, where R₆ represents H, NRcRd and Rc and Rd are independently H, C₁₋₄ alkyl group including branched alkyl optionally substituted with up to three halogen atoms, CHO or CONRcRd;
n is 0 - 4
R₅ is H or C₁₋₄ alkyl group including branched alkyl optionally substituted with up to three halogen atoms.

Examples of aryl groups include phenyl and naphthyl. Examples of heteroaryl include 5 membered heteroaryl groups including pyrazole, triazole, furan, thiophene, thiazole, oxazole or 6 membered heteroaryl rings including pyridine, pyrimidine, pyrazine, pyridazine and triazine.

In a further aspect the at least one Menin inhibitor is a compound according to formula (A), wherein
- R₁: is -CH₂CF₃
- R₂: is H or MeNH-
- R₃: is H or OH
- R₄: independently represents H, a 4-pyrazolyl group (I) or a substituted bicyclic alkyl group
- (II): 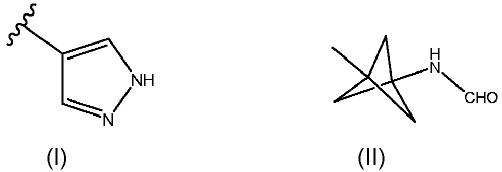
- n: is 0 - 4
- R₅: is H or methyl.

In a preferred aspect the at least one Menin inhibitor is a compound according to formula (A), wherein
- R₁: is -CH₂CF₃
- R₂: is H
- R₃: is H or OH
- R₄: is H or a 4-pyrazolyl group -
- n: is 0 or 1 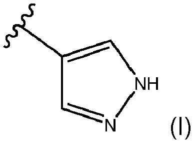
- R₅: is H or methyl.

In an even more preferred aspect, the at least one Menin inhibitor is a compound according to formula (A), wherein
- R₁: is -CH₂CF₃
- R₂: and R₅ are H
- R₃: is OH
- R₄: is a 4-pyrazolyl group - 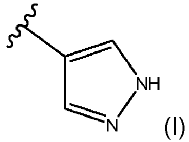
- n: is 1.

In one embodiment of the present invention the Menin inhibitor is selected from the group consisting of MI-538, MI-503, MI-463, MI-1481, MI-3454 or MI-2, or a combination thereof. Preferably the Menin inhibitor of present invention is MI-538, MI-503, MI-463 or MI-3454, most preferred MI-538.

The inhibitors MI-538, MI-503, MI-463, MI-1481 and MI-3454 have the following structures and can all be commercially obtained under these numbers:

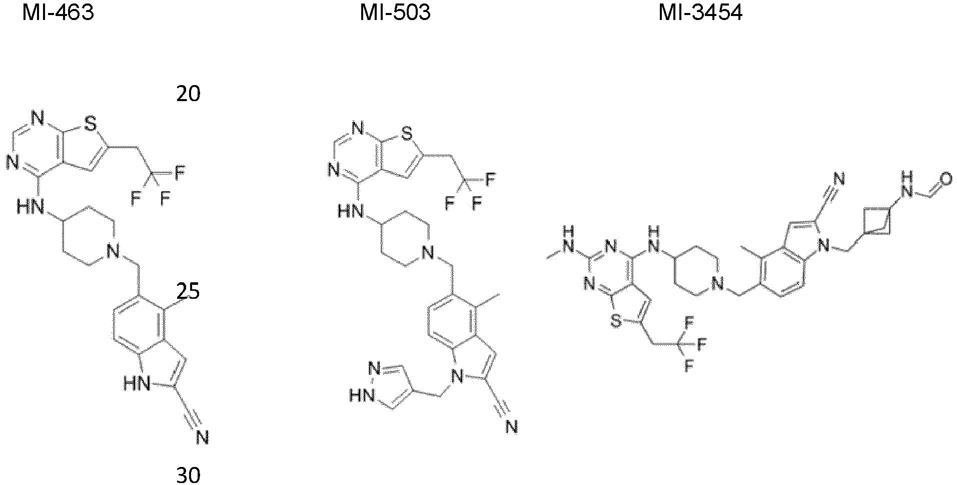

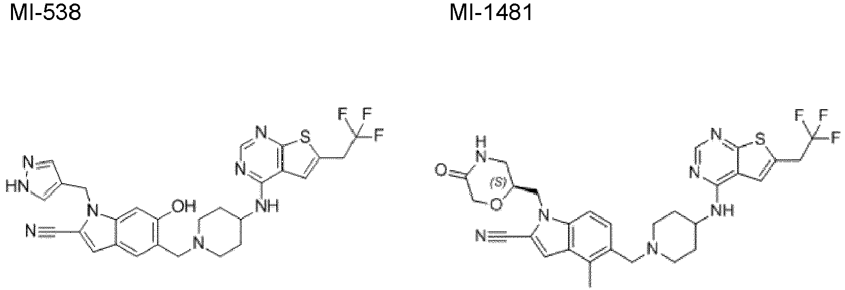

As shown in the Examples the addition of a class I HDAC inhibitor leads to a surprising enhancement of the effect of the Menin inhibitors. Without being bound by theory, the effect can be explained by the class I HDAC inhibitor modifying the binding of HDAC7 to its cofactors whilst the Menin inhibitor induces HDAC7. The class I HDAC inhibitor thus promotes that HDAC7 can exert its leukemia suppressor function.

In a further embodiment of present invention, the composition therefore further comprises at least one Class I HDAC inhibitor. The at least one Class I HDAC inhibitor can be selected from the group consisting of Chidamide, Entinostat, Mocetinostat, Tacedinaline or Romidepsin, or a combination thereof. A preferred Class I HDAC inhibitor of present invention is Chidamide.

In a preferred embodiment of present invention, the at least one Menin inhibitor is MI-538 and the at least one Class I HDAC inhibitor is Chidamide.

In some embodiments, the Menin inhibitor and the class I HDAC inhibitor are co-administered concurrently (e.g., simultaneously, essentially simultaneously or within the same treatment protocol) or sequentially.

In some embodiments, the Menin inhibitor and class I HDAC inhibitor are co-administered in separate dosage forms. In some embodiments, the Menin inhibitor and a class I HDAC inhibitor are co administered in combined dosage forms.

The present invention further relates to a kit of parts comprising at least two recipients, wherein one recipient comprises at least one Menin inhibitor and the other recipient comprises at least one Class I HDAC inhibitor.

In one embodiment the kit of parts comprises at least two recipients, wherein one recipient comprises at least one Menin inhibitor selected from the group consisting of MI-538, MI-2, MI-503, MI-463, MI-1481 or MI-3454, or a combination thereof, preferably wherein the Menin inhibitor is MI-538, MI-503, MI-463, or MI-3454, most preferred MI-538, and the other recipient comprises at least one Class I HDAC inhibitor selected from the group consisting of Chidamide, Entinostat or Mocetinostat, preferably wherein the at least one Class I HDAC inhibitor is Chidamide.

In one preferred embodiment of present invention the kit of parts comprises at least two recipients, wherein one recipient comprises MI-538 and the other recipient comprises Chidamide.

The present invention furthermore relates to the composition as disclosed herein above for use in a method of treatment of B cell acute lymphoblastic leukemia (B-ALL).

Other malignancies related to B lymphocytes in which HDAC7 has tumor suppressor functions, apart from pro-B-ALL, are also envisaged for indications that can be treated as described herein. For example, patients diagnosed of Diffuse Large B Cell Lymphoma or B-ALL in adults can also benefit from the treatment shown herein. In addition, HDAC7 plays an important role in T-lymphocytes and its induction can also contribute to a better treatment in malignancies derived from T cells.

The present invention therefore furthermore relates to the composition as disclosed herein above for use in a method of treatment of B-lymphocyte related malignancies or Diffuse Large B cell Lymphoma (DLBCL), follicular lymphoma (FL) and T-cell acute lymphoblastic leukemia (T-ALL), preferably B cell acute lymphoblastic leukemia (B-ALL).

The present invention furthermore relates to a method for treating B-lymphocyte related malignancies or Diffuse Large B cell Lymphoma (DLBCL), follicular lymphoma (FL) and T-cell acute lymphoblastic leukemia (T-ALL), preferably B cell acute lymphoblastic leukemia (B-ALL) in a subject in need thereof, comprising administering to said subject the composition as defined herein above.

As used herein the term "B cell acute lymphoblastic leukemia", in short "B-ALL", as used in present invention, refers to acute lymphoblastic leukemia (ALL), a blood cancer that develops from lymphocytes, also called acute lymphocytic leukemia, that develops from B cells.

The term "malignant hematologic cells" as used herein refers to hematologic cancer cells that have their origin in blood-forming tissue, such as the bone marrow, or in the cells of the immune system. Examples of hematologic cancer are leukemia, lymphoma, and multiple myeloma. Hematologic cancer is also called blood cancer.

The term "t(4;11) rearrangement" as used herein refers to a chromosomal anomaly involving a translocation between chromosomes 4 and 11. The chromosomal anomalies are designated by the prefix defining the type of anomaly, in the present case a "t" for translocation. The numbers in brackets indicate the chromosomes involved, i.e. chromosomes 4 and 11 (see e.g. Meyer et al. Leukemia 2006; 20:777-784).

The term "Menin Inhibitor" as used herein relates to inhibitors of Menin, a protein encoded by the *MEN1* gene. Menin is a tumor suppressor associated with multiple endocrine neoplasia type 1 (MEN-1 syndrome).

The terms "enhance" or "enhancing" as used herein mean to increase or prolong either in potency or duration a desired effect. By way of example, "enhancing" the effect of therapeutic agents refers to the ability to increase or prolong, either in potency or duration, the effect of therapeutic agents during treatment of a disease, disorder or condition.

The term "Class I HDAC inhibitor" as used herein refers to inhibitors of class I histone deacetylases (HDACs). Histone acetylation levels are maintained by dynamic actions of histone acetyltransferases (HATs), responsible for acetylation, and HDACs, responsible for deacetylation. The HDAC family can be divided into four major classes, each of which consists of different HDAC members: class I (including HDAC 1, 2, 3 and 8), class II (including HDAC 4, 5, 6, 7, 9, 10), class III (including SIRTs 1-7) and class IV (HDAC 11).

It is finally contemplated that any features described herein can optionally be combined with any of the embodiments of any method, medical use, kit and use of a kit of the invention; and any embodiment discussed in this specification can be implemented with respect to any of these. It will be understood that particular embodiments described herein are shown by way of illustration and not as limitations of the invention.

All publications and patent applications are herein incorporated by reference to the same extent as if each individual publication or patent application was specifically and individually indicated to be incorporated by reference.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

### EXAMPLES

### MATERIALS AND METHODS

### Cell cultures

Human SEM-K2, RS4;11 and REH cell lines were grown and maintained in regular cell culture conditions (RPMI 1640 medium + 10% fetal bovine serum), Where indicated, cells were treated with the respective Menin inhibitor, e.g.MI-463, MI-503, MI-538 or MI-3454, (1 µM, 6 days) and/or Chidamide (1 µM, 48 h). Genetically modified SEM-K2 cells (SEM-TetOn-Tight-HDAC7) were also cultured in RPMI 1640 medium + 10% fetal bovine serum conditions, together with G418 (1.5 mg/mL) and puromycin (0.4 µg/mL) to maintain antibiotic selection. For prednisone treatment experiments, HDAC7 was induced with 1 µg/mL doxycycline 24 h before treating cells with prednisone at 10 µg/mL or 100 µg/mL doses, which was maintained for further 48 h.

### RNA extraction and RT-qPCR expression analysis

Total RNA from SEM-K2 cells samples was extracted using Trizol (Life Technologies, Thermo Fisher), following the manufacturer's instructions. After being quantified, 1-2 µg of RNA were retrotranscribed with random hexamers using a High-Capacity cDNA Reverse Transcription kit (Applied Biosystems, Thermo Fisher). mRNA levels were determined by qRT-PCR using Light Cycler 480 SYBR Green Master Mix (Roche) either in a Light Cycler 480 II (Roche) or in a QuantStudio 7 Flex Real-Time PCR (Applied Biosystems) apparatus. Results were analyzed using LightCycler 480 software (Roche), taking *GAPDH* and *RPL38* as housekeeping genes. All primer pairs were designed with Primer3^{®} software.

### Protein extraction and western blot

For protein extraction, SEM-K2, RS4;11 and REH cells were washed with ice-cold PBS and resuspended in RIPA lysis buffer (150 mM NaCl, 50 mM Tris-HCI pH 8.0, 1% NP40, 0.1% SDS, 0.5% sodium deoxycholate) containing Complete Mini EDTA-free protease inhibitors cocktail (Roche), PMSF and DTT. Lysates were sonicated in a UP50H ultrasonic processor (Hielscher), clarified by maximum speed centrifugation and quantified with Bradford reagent (Bio-Rad). Protein lysates were boiled and loaded onto 10% polyacrylamide gels and transferred to 0.2-µm nitrocellulose membranes (Amersham Protran, GE Healthcare Lifescience). After blocking with 5% non-fat milk, membranes were blotted with antibodies against HDAC7 (clone H-273, Santa Cruz Biotechnologies; 1:500) and β-actin (clone AC-15, Sigma-Aldrich, 1:2000) overnight at 4°C. After washing with TBS-Tween, membranes were incubated with HRP-conjugated secondary antibodies (anti-mouse HRP and anti-rabbit HRP; 1:3000) for 1-2 h at r.t. The reaction was developed with the ECL Western Blotting Analysis System (Amersham). Images shown are representative of at least three independent experiments.

### MTT cell viability assays

For MTT assays, 5 x 10⁴ SEM-K2, RS4;11 and REH cells were seeded in 12-well plates, in triplicate for each condition tested. After the corresponding drug exposition time (MI-463, MI-503, MI-538, MI-3454, Chidamide or Prednisone) MTT reagent was added at a final concentration of 5 mg/mL. Cells were incubated for 3 h under regular conditions and, afterwards, the resulting formazan blue product was solubilized in DMSO. Absorbance was measured as the difference between absorbance at 570 nm and background absorbance measured at 750 nm. Data are presented as the average of at least three independent experiments, each one of them performed in triplicate.

### Example 1: Upregulation of HDAC7 at protein and mRNA level

### A) Treatment of SEM-K2 and RS4;11 cell lines with MI-538 and/or Chidamide

SEM-K2 and RS4;11 cell lines (both harboring t(4;11) translocation) were treated with MI-538 and/or Chidamide, or DMSO as control. β-actin was used as loading control. Protein levels of HDAC7 in SEM-K2 and RS4;11 cells were determined. The results can be seen in Fig 1A, where the protein levels of HDAC7 in SEM-K2 (left panel) and RS4;11 (right panel) are shown.

### B) qRT-PCR for HDAC7 in SEM-K2 cells

SEM-K2 cells were treated with MI-538 and/or Chidamide, or DMSO as control. GAPDH and RPL38 were used as housekeeping genes. Error bars represent SE. Statistical significance is indicated as: * p < 0.05; **, p < 0.01; ***, p < 0.001; or n.s., non-significant.

A clear upregulation of HDAC7 at protein levels in both cell lines (Figure 1A) and also at mRNA level in SEM-K2 cells (Figure 1B) could be observed. As can be seen from Figure 1B, in SEM-K2 cells, treatment with class I HDAC selective inhibitor Chidamide does not provide any increase in mRNA expression of HDAC7, whereas treatment with MI-538 alone leads to an about 1,5 fold increase of mRNA expression of HDAC7 in the cells. Surprisingly, the combination of Chidamide and Menin inhibitor MI-538 provides a significant increase in comparison to the use of Chidamide or MI-538 alone in SEM-K2 cells.

As expected, Chidamide did not alter HDAC7 levels in SEM-K2 cells, but slightly increased its expression in RS4;11 (Figure 1A). The combination of both drugs triggered HDAC7 mRNA in SEM-K2 cells (Figure 1B), converting these combination in a promising strategy for further studies aiming to reduce t(4;11) pro-B-ALL cells leukemogenic capacity.

### C) Quantification of expression of HDAC7 protein

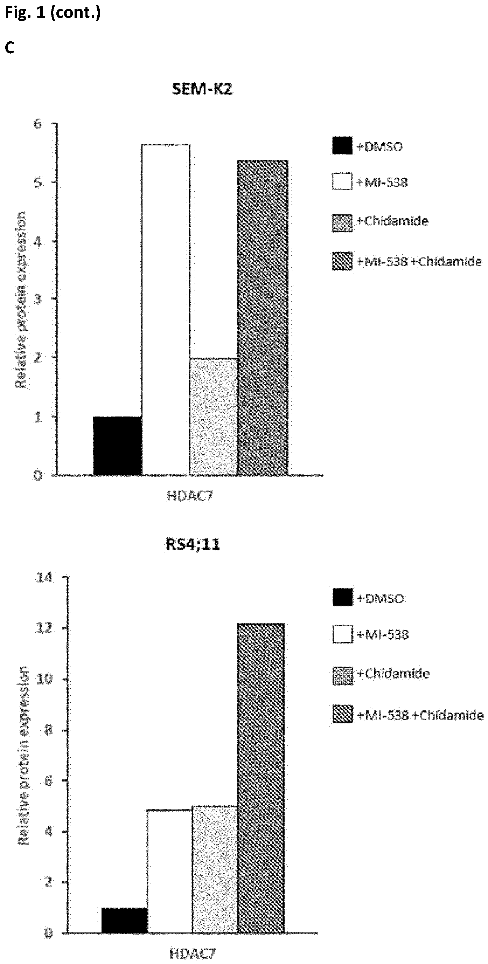

The expression of HDAC7 protein in Figure 1A has been quantified and can be seen in Figure 1 C.

### Example 2: MTT viability assays

MTT viability assays were conducted in a panel of three pro-B-ALL cell lines, including previously used SEM-K2 and RS4;11, together with REH cells, that harbor an unaltered form of *MLL* gene. As expected, REH cells displayed abundant levels of HDAC7, compared to t(4;11) translocated cells (Figure 2A). MI-538 strongly reduced viability of both SEM-K2 and RS4;11 cells, but its combination with Chidamide produced an even more dramatic effect, being significantly stronger than both drugs administered alone (Figure 2B, left and center panels). Interestingly, single therapy with Chidamide presented a low effect in SEM-K2 cells, whereas cell viability reduction was similar to that caused by MI-538 in RS4;11 cell line. This data correlates with the induction of HDAC7 provoked by Chidamide in this specific cell line. The demonstration that the effect of these drugs is, at least partially, a consequence of HDAC7 induction can be extracted from the MTT assays performed in REH cell line. This cell line, with strong expression of HDAC7, did not respond to MI-538 and/or Chidamide treatments, and no effect was observed on cell viability (Figure 2B, right panel).

### Example 3: Expression of key HDAC7 targets after induction of HDAC7 with MI-538 and Chidamide

A forced overexpression of HDAC7 in SEM-K2 shifts genomic profile of t(4;11) pro-B-ALL cells towards a more anti-oncogenic profile through the altered expression of targets involved in key hallmarks of cancer, such as cell differentiation, chemotherapy resistance or apoptosis prevention. In order to corroborate whether the effect of MI-538 and Chidamide on endogenous HDAC7 can be paralleled to that of exogenous HDAC7 induction, we analysed some of HDAC7 targets previously reported. qRT-PCR was performed for *MMP9* (involved in apoptosis), CD86 (marker of proper B lymphocytes differentiation) and *ASNS* (chemoresistance marker in infant leukemia) in SEM-K2 cells treated with MI-538 and/or Chidamide, or DMSO as control. Error bars represent SE. Statistical significance is indicated as: * p < 0.05; **, p < 0.01; ***, p < 0.001; or n.s., non-significant.

According to what the inventors expected, both MI-538 and Chidamide triggered the mRNA levels of the pro-apoptotic gene *MMP9* and the marker of B lymphocyte differentiation CD86, showing a synergistic effect when both drugs were administered together (Figure 3, left and center panels). Oppositely, the chemoresistance marker *ASNS* is downregulated upon treatment of SEM-K2 cells with MI-538 and Chidamide (Figure 3, right panel), according to previous data showing an opposite pattern of expression with HDAC7 (de Barrios et al. 2021). Therefore, the effect of MI-538 and Chidamide not only affects HDAC7 itself, but also its previously described targets, reinforcing the potential use of this combinatorial therapy to improve outcome of t(4;11) pro-B-ALL.

### Example 4: Effect of HDAC7 expression on standard therapy

As a proof-of-concept, the effect of HDAC7 expression was evaluated on the regular and standard therapy that is commonly used in clinical settings in infant t(4;11) pro-B-ALL patients. SEM-TetOn-Tight-HDAC7 cell line (also used in de Barrios et al. 2021), that permits HDAC7 induction with doxycycline treatment was used. As shown in Figure 4, HDAC7 induction endows SEM-K2 cells with a higher sensitivity to prednisone treatment, since the reduction of viability is significant at a 10-time lower dose than in normal conditions (i.e. with low HDAC7 levels).

### Example 5: MTT viability assays with Menin Inhibitors MI-3454, MI-503 and MI-463

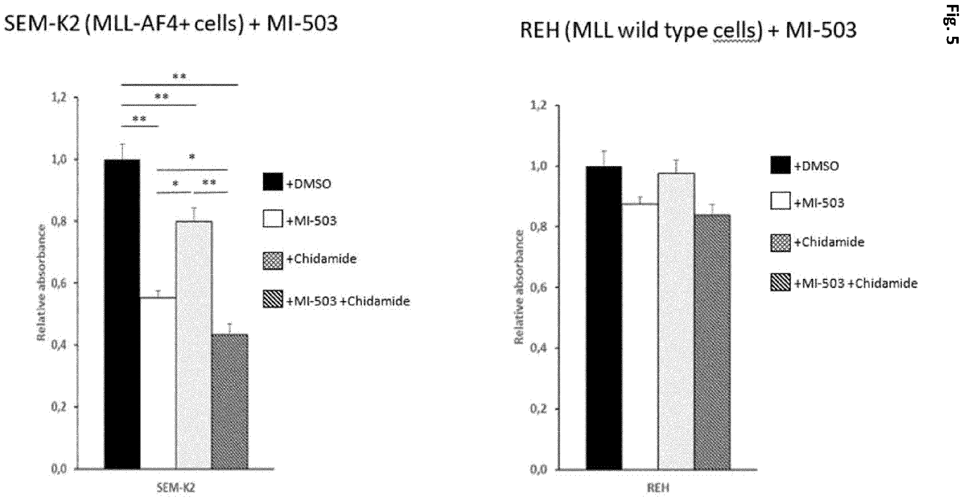

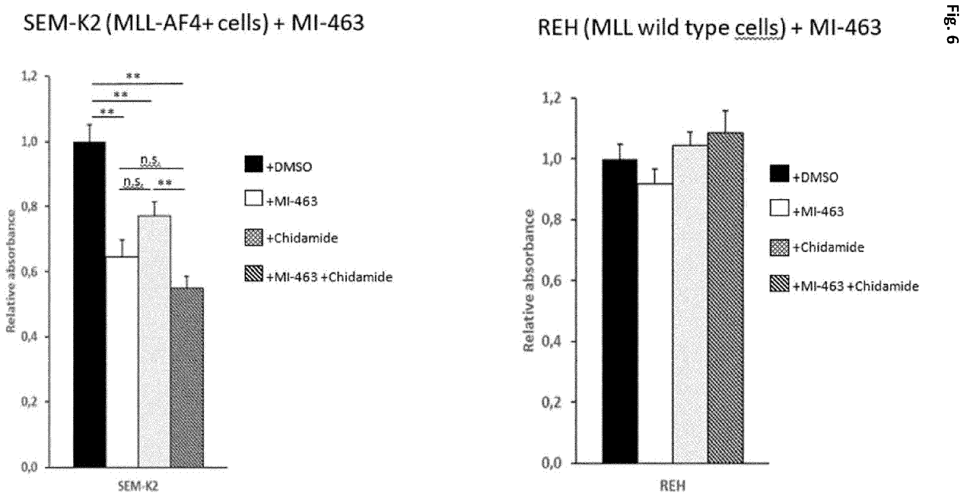

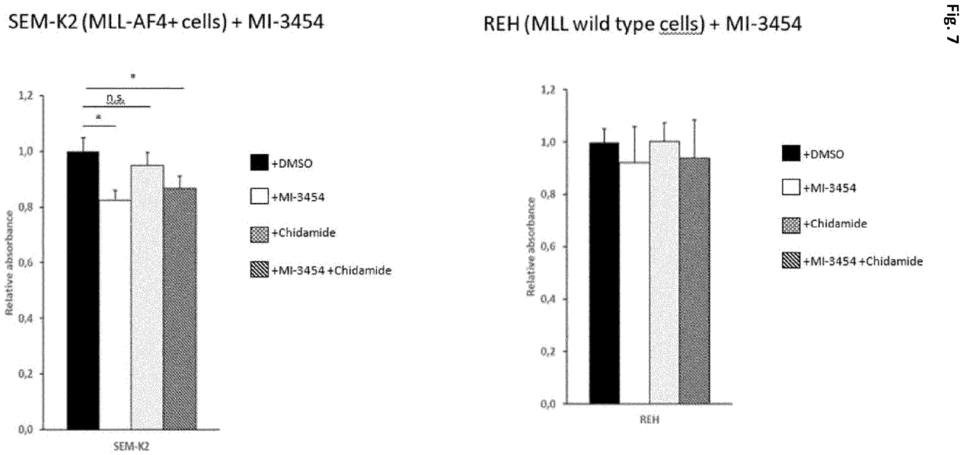

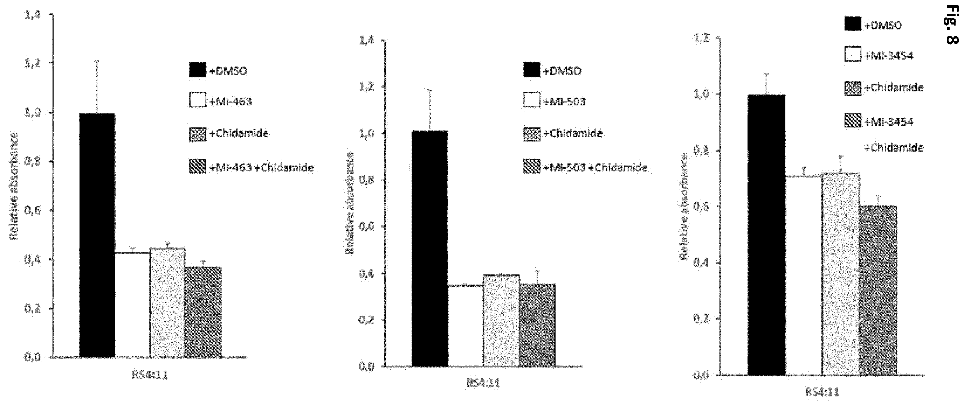

As described in Example 2, the same MTT viability assays were also conducted for other Menin inhibitors MI-3454, MI-503 and MI-463. The results are shown in Fig.5-8. As can be seen in Fig.5, MI-503 shows a strong effect on SEM-K2 cells and the combination with Chidamide shows an even stronger effect, whereas REH cells show only a very weak response to this drug. Fig.6 shows the effect of MI-463, that reduces viability of SEM-K2 cells. However, no additional effect is observed when treated with Chidamide. As expected REH cells do not respond to this inhibitor. Fig.7 shows an effect of Menin inhibitor MI-3454, albeit weaker than the one of MI-503 and MI-463. As can be observed in Fig.8, the use of each of the three Menin inhibitors MI-3454, MI-503 and MI-463 shows an effect on RS4;11 cells, in the case of inhibitor MI-3454 the effect is weaker, but statistically significant.

### Example 6: mRNA expression of HDAC7 in SEM-K2 and REH cell lines

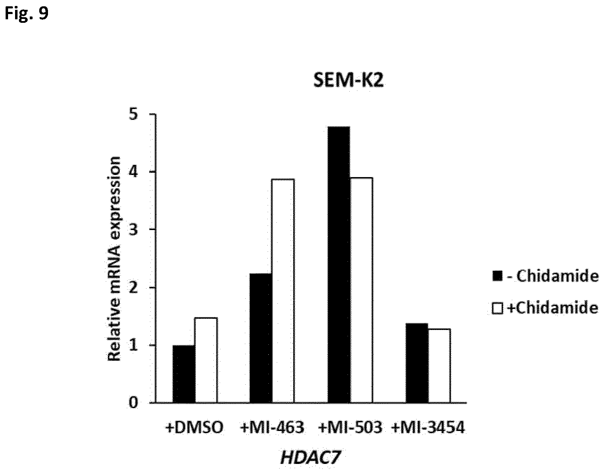

mRNA expression of HDAC7 in SEM-K2 and REH cell lines has been assessed according to the protocol as described in material the material and methods part. As can be seen in Fig.9 HDAC7 expression increases when SEM-K2 cells are treated with MI-503, MI-463 and MI-3453 (with or without Chidamide).

### Example 7: HDAC7 protein expression in SEM-K2 cells

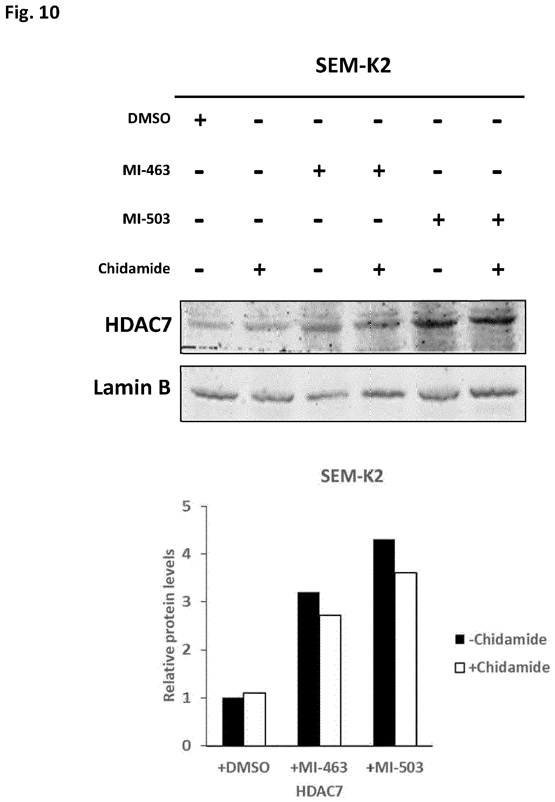

HDAC7 protein expression in SEM-K2 cells has been assessed according to the protocol as described in material the material and methods part. As can be seen in Fig.10, protein expression of HDAC7 is triggered by MI-503 and MI-463.

## Claims

1. A composition, preferably a pharmaceutical composition, for use in a method of inducing expression of HDAC7 in malignant hematologic cells, wherein the composition comprises at least one Menin inhibitor.

2. The composition for use according to claim 1, wherein the at least one Menin inhibitor is a compound according to formula (A) 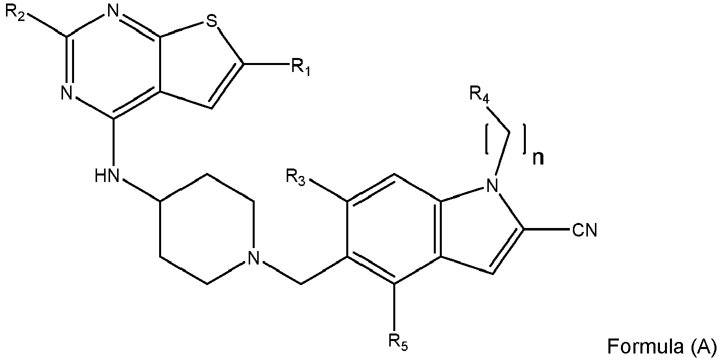 wherein
R₁ is a C₁₋₄ alkyl group including branched alkyl optionally substituted with up to three halogen atoms;
R₂ is H or NRaRb, Ra and Rb are independently H or C₁₋₄ alkyl;
R₃ is H or OH;
R₄ independently represents H, C₁₋₄ alkyl group including cycloalkyl, or aryl group including heteroaryl;
R₄ is optionally substituted with R₆, where R₆ represents H, NRcRd and Rc and Rd are independently H, C₁₋₄ alkyl group including branched alkyl optionally substituted with up to three halogen atoms, CHO or CONRcRd
n is 0 - 4
R₅ is H, or a C₁₋₄ alkyl group including branched alkyl optionally substituted with up to three halogen atoms.

3. The composition of claim 2, wherein
R₁ is -CH₂CF₃
R₂ is H or MeNH-
R₃ is H or OH
R₄ independently represents H, a 4-pyrazolyl group (I) or a substituted bicyclic alkyl group (II) 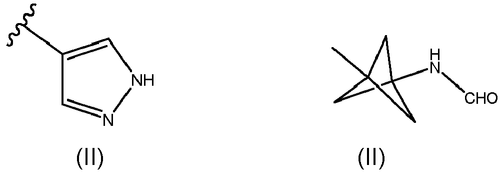 n is 0 - 4
R₅ is H or methyl.

4. The composition of claims 1 to 3, wherein
R₁ is -CH₂CF₃
R₂ is H
R₃ is H or OH
R₄ is H or a 4-pyrazolyl group - 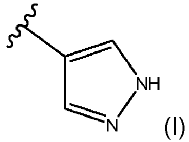
n is 0 or 1
R₅ is H or methyl.

5. The composition of claims 1 to 4, wherein
R₁ is -CH₂CF₃
R₂ and R₅ are H
R₃ is OH
R₄ is a 4-pyrazolyl group - 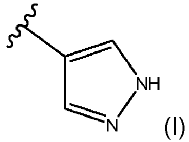
n is 1.

6. The composition for use according to any one of claims 1 to 5, wherein the at least one Menin inhibitor is selected from MI-538, MI-503, MI-463, MI-3454, preferably wherein the Menin inhibitor is MI-538.

7. The composition for use according to any one of claims 1 to 6, wherein the expression of HDAC7 is mRNA expression and wherein said mRNA expression of HDAC7 is increased by at least about 1.2-fold, 1.3-fold, 1.4-fold, 1.5-fold in the malignant hematologic cells compared to the mRNA expression of HDAC7 in reference cells.

8. The composition for use according to any one of claims 1 to 7, wherein the expression of HDAC7 is protein expression and wherein said protein expression of HDAC7 is increased by at least about 1.5-fold, 2.0-fold, 2.5-fold, 3.0-fold, 3.5-fold, 4.0-fold, 4.5-fold, 5.0-fold, 5.5-fold, 6.0-fold, 6.5-fold, 7.0-fold, 7.5-fold, 8.0-fold, 8.5-fold, 9.0-fold, 9.5-fold, 10.0-fold, 10.5-fold, 11.0-fold, 11.5-fold, 12.0-fold in the malignant hematologic cells compared to the protein expression of HDAC7 in reference cells.

9. The composition for use according to any one of claims 1 to 8, wherein the malignant hematologic cells are selected from B cell acute lymphoblastic leukemia (B-ALL) cells, diffuse large B cell lymphoma (DLBCL) cells and follicular lymphoma (FL), preferably wherein the B-ALL cells are B cell acute lymphoblastic leukemia (B-ALL) cells.

10. The composition for use according to any one of claims 1 to 9, wherein the B-ALL cells are pro-B-all cells **characterized by** a t(4;11) rearrangement.

11. The composition for use according to any one of claims 1 to 10, wherein the composition further comprises at least one Class I HDAC inhibitor.

12. The composition for use according to claim 11, wherein the at least one Class I HDAC inhibitor is selected from the group consisting of Chidamide, Entinostat, Mocetinostat, Tacedinaline and Romidepsin, or a combination thereof.

13. The composition for use according to claim 12, wherein the at least one Class I HDAC inhibitor is Chidamide.

14. The composition for use according to any of claims 11 to 13, wherein the at least one Menin inhibitor is MI-538 and the at least one Class I HDAC inhibitor is Chidamide.

15. A kit of parts comprising at least two recipients, wherein one recipient comprises at least one Menin inhibitor and the other recipient comprises at least one Class I HDAC inhibitor according to any one of the preceding claims.

16. The kit of parts according to claim 15, wherein the at least one Menin inhibitor is selected from the group consisting of MI-538, MI-503, MI-463 or MI-3454, preferably wherein the at least one Menin inhibitor is MI-538.

17. The kit of parts according to any of claims 15 or 16, wherein the at least one Class I HDAC inhibitor is selected from the group consisting of Chidamide, Entinostat Mocetinostat, Tacedinaline and Romidepsin, or a combination thereof, preferably wherein the at least one Class I HDAC inhibitor is Chidamide.

18. The kit of parts according to any of claims 15 to 17, wherein the at least one Menin inhibitor is MI-538 and the at least one Class I HDAC inhibitor is Chidamide.

19. The composition according to any one of claims 1 to 14 and the kit of parts of claims 15 to 18 for use in a method of treatment of B cell acute lymphoblastic leukemia (B-ALL), diffuse large B cell lymphoma (DLBCL) or follicular lymphoma (FL), preferably of B cell acute lymphoblastic leukemia (B-ALL).

20. A method for treating B cell acute lymphoblastic leukemia (B-ALL), diffuse large B cell lymphoma (DLBCL) or follicular lymphoma (FL), preferably B cell acute lymphoblastic leukemia (B-ALL) in a subject in need thereof, comprising administering to said subject the composition as defined in claims 1 to 14.
